(19) **Europäisches Patentamt**

**European Patent Office**

**Office européen des brevets**

(11) Publication number: **0 296 845 B1**

(12) # EUROPEAN PATENT SPECIFICATION

(45) Date of publication of patent specification: **12.08.92**

(51) Int. Cl.⁵: **A61K 31/71**, A61K 47/44, A61K 9/10

(21) Application number: **88305718.4**

(22) Date of filing: **22.06.88**

The file contains technical information submitted after the application was filed and not included in this specification

(54) **Drug emulsion.**

(30) Priority: **23.06.87 GB 8714652**

(43) Date of publication of application:
**28.12.88 Bulletin 88/52**

(45) Publication of the grant of the patent:
**12.08.92 Bulletin 92/33**

(84) Designated Contracting States:
**AT BE CH DE ES FR GB GR IT LI LU NL SE**

(56) References cited:
**EP-A- 0 202 837**
**EP-A- 0 211 258**
**EP-A- 0 215 313**
**WO-A-82/01821**

**Int. J. Pharmaceutica, 46 (1988), p. 25-30**

**Martindale, The Extra Pharmacopoeia, 1982**

(73) Proprietor: **The University of Nottingham**
**University Park, University Boulevard**
**Nottingham NG7 2RD(GB)**

(72) Inventor: **Davis, Stanley Stewart**
**5 Wortley Hall Close**
**Universitiy Park, Nottingham(GB)**
Inventor: **Washington, Clive**
**15 Midland Avenue**
**Stapleford, Nottingham(GB)**

(74) Representative: **Bassett, Richard Simon et al**
**ERIC POTTER & CLARKSON St. Mary's Court**
**St. Mary's Gate**
**Nottingham NG1 1LE(GB)**

EP 0 296 845 B1

## Description

The antibiotic amphotericin B is extremely beneficial in certain infectious conditions, particularly those caused by the fungal organism Candida. The existing therapy is in the form of a product called Fungizone (Regd. T.M., Squibb) which consists of a solubilised formulation of amphotericin in the natural surfactant material sodium deoxycholate. This product is marketed by the Squibb Company. While helpful in combating Candida infections this product is not without its adverse reactions and side effects. It has been shown clearly that the Fungizone formulation can have a toxic effect particularly towards the kidney (see for example Reynolds et al (1963), Med. Clin. North American 47 1149-1154). The antibiotic properties of amphotericin are due to its binding to sterols in cell membranes and the subsequent formation of a membrane pore. The binding to ergosterol, the primary fungal sterol, is stronger than the binding to the mammalian sterol cholesterol. Hence the toxicity of amphotericin is only selective for fungal cells and not specific; this is the origin of the side effects in patients. Alternative strategies for administering amphotericin have been investigated and work conducted in Texas by Juliano, Lopez-Berenstein and others is particularly noteworthy (see for example Mehta et al (1984), Biochem. Biophys. Acta 770 230-234). These workers have employed a liposome formulation (phospholipid vesicle) in order to achieve benefit in terms of therapy. Others working along similar lines include the Squibb Company itself with the pro-liposome concept (see for example Payne et al (1987), J. Pharm, Pharmacol. 39 24-28). While the liposomal system might be beneficial clinically it is well known that liposomes are normally difficult to prepare and can be unstable.

While it is possible to produce an amphotericin emulsion system by the simple admixture of a commercial fat emulsion product (Intralipid, (Regd. T.M., Kabi) with the commercial solubilised system of amphotericin (Fungizone) (see, for example, EP-A-202 837), this system is unstable in that it produces a precipitate of the drug after this admixture and also has poor stability if stored for more than a few hours. The amphotericin B apparently is not intercalated at the oil-water interface in the additive formulations.

EP-A-215 313 (American Cyanamid) discloses certain emulsions which break easily on administration to a patient. The drug is mixed with an oil phase before water is added to form an emulsion. Benzyl alcohol is used as a co-surfactant. The emulsions not only break quickly on administration but are not very stable in storage.

WO 82/01821 (Chinoin) discloses formulations which have the drug as a solid suspension dispersed throughout an emulsion. Again, the emulsions are not very stable and do not overcome the problem of the toxicity of the drug; it is to be noted that all the prior formulations are for topical application.

EP-A-211258 (Abbott) discloses certain microemulsions, which are quite distinct from the emulsions of the present invention.

It is the intention that the present invention should provide an improved formulation for amphotericin B and other drugs with which similar difficulties have been experienced.

One aspect of the present invention provides an oil-in-water surfactant-stabilised emulsion of a drug, the drug being poorly soluble in both oil and water, characterised in that at least 50% of the drug is present in the surfactant layer.

The location of the drug in the formulation can be established by known techniques, such as fluorescence quenching, using a suitable marker compound known to locate itself in the surfactant layer. In the case of amphotericin B, which has an intense structured ultraviolet absorption in the range 300-400nm, pyrene is a suitable marker. So that only quenching in the surfactant layer is observed, a pyrene-labelled phospholipid may be used, for example B-(pyrene-1-yl)-decanoyl-gamma-palmitoyl-1-alpha-phosphatidylcholine, available from Sigma.

Preferably, at least 60%, 70%, 80%, 90% or 99% of the drug is in the surfactant, or interfacial, layer. Most preferably, substantially all of the drug is in the surfactant layer.

A "poorly soluble" drug is one which is insufficiently soluble for therapeutic levels to be achieved by the administration of a convenient volume of solution. In terms of an infusion of a formulation containing the drug, it would generally be the case that one would wish to administer less than 100 ml of the formulation per hour, preferably less than 50 ml/hour, more preferably less than 30 or 10 ml/hour. In essence, the formulations of the invention are particularly suitable for drugs which would be categorised in pharmacopoeias as "practically insoluble" in water or oils. In addition, the drug should have enough amphipathic character to be retained in the surfactant layer; a material such as silica is insoluble in water or oil but equally is not sufficiently amphipathic to be located selectively in the surfactant layer. Such an inert compound is, however, unlikely to have any pharmacological activity. The man skilled in the art will readily be able to determine by routine and non-inventive experiments whether a drug is suitable.

The drug may be a general anaesthetic, local anaesthetic, hypnotic, sedative, autacoid or autacoid antagonist (for example a prostaglandin), antibiotic or antimicrobial, antineoplastic (especially cytotoxic drugs such as methotrexate) or immunosuppressant. A particularly preferred group of drugs is the polyene antibiotics including tetraenes such as nystatin, pentaenes such as aliomycin, methylpentaenes such as filipin, carbonylpentaenes such as mycoticin, hexaenes such as cryptocidine, carbonylhexaenes such as dermostatin, and heptaenes such as amphotericin B. Such antibiotics are commercially available or can be conventionally prepared by techniques known to one of skill in the art. Preferably, the said drug is amphotericin B, nystatin or filipin, most preferably amphotericin B.

The surfactant may be a Pluronic® (Regd. T.M.) compound such as Pluronic® F68, or 127, or Tetronic® or any other suitable non-ionic surfactant but is preferably a phospholipid, most preferably a lecithin or purified phosphatidyl choline.

A second aspect of the invention provides a process for preparing a drug-containing emulsion, comprising the steps of (a) optionally dissolving the drug in a suitable co-solvent, (b) mixing the drug or a solution thereof in the co-solvent with an aqueous preparation of a surfactant, (c) removing at least most of any co-solvent which is present, (d) mixing the product of step (c) with oil, and (e) forming an oil-in-water emulsion from the product of step (d).

An important feature of the process for preparing the emulsion of this invention is that the drug is solubilised in the surfactant before the emulsion is formed. Thus, the emulsion may be prepared by dissolving the drug in a suitable solvent, mixing the resulting solution with an aqueous dispersion or solution of the surfactant, evaporating off most or all of the solvent and forming an emulsion with oil. If the drug is suitable (which amphotericin is not), the drug may simply be shaken etc with the surfactant solution, and the cosolvent may not be necessary. A co-solvent is preferred.

The solvent (if used) will be selected by reference to the properties of the drug, but is preferably volatile and is conveniently a lower alkanol, an ether or a ketone, most preferably methanol. The term "volatile" is using herein to describe any solvent which may satisfactorily be evaporated off in step (c) of the process at a temperature which will not destroy the drug or evaporate the water. A solvent of boiling point 80°C or less is frequently suitable, but others may be suitable, depending upon the latent heat of vaporisation of the solvent. Suitably, the solvent should be at least slightly hydrophilic so that a homogeneous solution/dispersion is formed in step (b) of the process and so that any solvent which remains will not disperse in the oil phase of the final emulsion.

Any pharmaceutically acceptable oil may be used, for example soybean or safflower oil or medium chain triglycerides (such as miglyol®) or acetylated monoglycerides.

In the final emulsion, the proportion of oil is suitably 5 to 50%, conveniently 5 to 30%, preferably 5 to 20%.

The surfactant is used at any suitable level, for example 0.5 to 10%, preferably 1 to 3%, most preferably 1 to 2%.

The level of drug may be chosen by one skilled in the art to suit the dosage regimen and so on but may typically be up to 1 mg/ml, preferably about 0.5 mg/ml, in the case of Amphotericin B.

These percentages refer to the final emulsion, the remainder being made up of water.

The emulsion will normally be sterilised, for example by filtration or heat treatment, to render it suitable for parenteral delivery, and antioxidant, preferably lipophilic or amphiphilic such as tocopherol, may be added before sterilisation.

Emulsions in accordance with the invention can be administered topically, orally, rectally or by "aerosolisation" into the lungs, but will usually be administered parenterally, for example by continuous venous infusion or by injection, which may be intravenous, subcutaneous or intramuscular. Sustained release preparations such as subcutaneous depots may be used. The daily dose will be determined by the skilled man, with reference to the patient, the disease and the drug, but might typically be 0.10 mg/kg/day to 10 mg/kg day, total body weight.

In the case of the polyene antifungal drugs such as amphotericin B, the formulations of the invention are useful in the treatment of humans or animals suffering from a variety of fungal infections, for example caused by any species of Candida - (especially C. albicans and C. tropicalis), Torulopsis glabrata and Aspergillus spp. These infections are especially common, and serious, in immunocompromised patients, such as those treated with immunosuppressant drugs or those suffereing from Acquired Immunodeficiency Syndrome (AIDS; acute HIV infection). Formulations in accordance with the present invention have been found to provide three advantages. Firstly, through a novel method of formulation, it is possible to incorporate amphotericin and similar drugs into the surfactant layer of an oil-in-water emulsion to provide a drug loading that allows the system to be used clinically. Secondly, the drug is thought to be trapped (most likely intercalated into the surfactant layer of the emulsion) and has been found to be far less toxic to red blood cells than the commercially available systems. Thirdly, by the use of appropriate conditions and additives, it has been found possible to prepare a sterile formulation of the amphotericin emulsion by processes of filtration or terminal heat sterilisation.

A preferred embodiment of the invention will now be described by way of example and with reference to the accompanying drawings, in which:

Figure 1 shows the release of haemoglobin from erythrocytes exposed to compositions in accordance with the invention and to known compositions;

Figure 2 shows the extent of erythrocyte lysis resulting from exposure to simple, solubilised compositions (i.e. not in accordance with the invention);

Figure 3 is comparable with Figure 2, and illustrates the reduced cell lysis when emulsions in accordance with the invention are used;

Figure 4 shows the long term stability of the particle sizes in an emulsion in accordance with the invention;

Figure 5 shows the long term stability of the content of amphotericin B in an emulsion in accordance with the invention; and

Figure 6 shows the survival time of fungally-infected mice treated with compositions in accordance with the invention.

EXAMPLE 1: Preparation of the emulsion system

An intravenous emulsion with an amphotericin concentration of 0.5 mg/ml was prepared as follows:
(quantities for 100 ml emulsion).
50 mg Amphotericin B (Sigma) dissolved in 100 ml methanol.
1.2 g egg phosphatidylcholine (Lipoid; E80) dispersed in 90 ml water (Silverson blender).
200 mg cholesterol dissolved in 10 ml soya oil (for 2% added sterol).

The methanolic Amphotericin was mixed with the lecithin dispersion and briefly sonicated to aid mixing. The mixture was then evaporated to approximately 20 ml on a rotary evaporator to remove much of the methanol, and water added to a total of 150 ml. The mixture was again sonicated (probe, 80W, 5 minutes) to release methanol trapped in lipid vesicles and re-evaporated to a volume of approximately 50 ml on a rotary evaporator. This sequence is necessary to remove the maximum amount of methanol. The product was made up to 90 ml with water, 10 ml of oil was added and the whole sonicated in an ice bath (probe, 50W, 30 minutes) to produce the emulsion.

The amphotericin loading can be increased to 1 mg/ml by increasing the lecithin concentration to 1.8 g/100 ml emulsion and 20 ml soya oil/100 ml emulsion.

It should be noted that the amphotericin B solution in methanol should preferably be centrifuged before use to remove insoluble impurities and that the lecithin/water/methanol system is prone to foaming during evaporation; this tendency disappears as the methanol is removed. Other emulsifiers acceptable to regulatory agencies (GRAS status) can be used to prepare the emulsion. The poloxamer and poloxamine series (block copolymers of polyoxyethylene/polyoxypropylene obtained from BASF) can be employed, for example poloxamer F68, F127, L92.

Dextran (mw 500,000, 1 mg/ml) can be added to prevent the small extent of precipitation seen on autoclaving. Tonicity can be adjusted with an agent that does not affect emulsion stability (i.e. a non-electrolyte) nor one that enhances the solubility of amphotericin in the aqueous phase (not a polyhydroxylic liquid). An amino acid, such as glycine, alanine) is a suitable choice.

EXAMPLE 2: Reduced interaction against red blood cells

Lysis of human erythrocytes was measured by the method of Mehta et al op.cit.). The blood was donated from healthy males in the age range 19-30 years or from the local blood bank. It was centrifuged (5 minutes at 2500 g) to sediment erythrocytes, which were washed twice with isotonic saline. The final washing showed no detectable haemoglobin absorbance. Blood and washed erythrocytes were stored at 4°C and used within 1 week of donation.

Washed erythrocytes were suspended in isotonic saline to an absorbance of 2.0 at 550 nm. The amphotericin formulations were added to produce final amphotericin concentrations in the range 0-32 ug/ml. These were vortex mixed and incubated at 37°C for 20 minutes. The tubes were then centrifuged for 10 minutes at 3000 g and the clear supernatant assayed for haemoglobin by absorbance at 550 nm. The mixtures containing the emulsion formulation showed considerable droplet scattering, but haemoglobin could be assayed (UV, 550 nm) when diluted by a factor of 10.

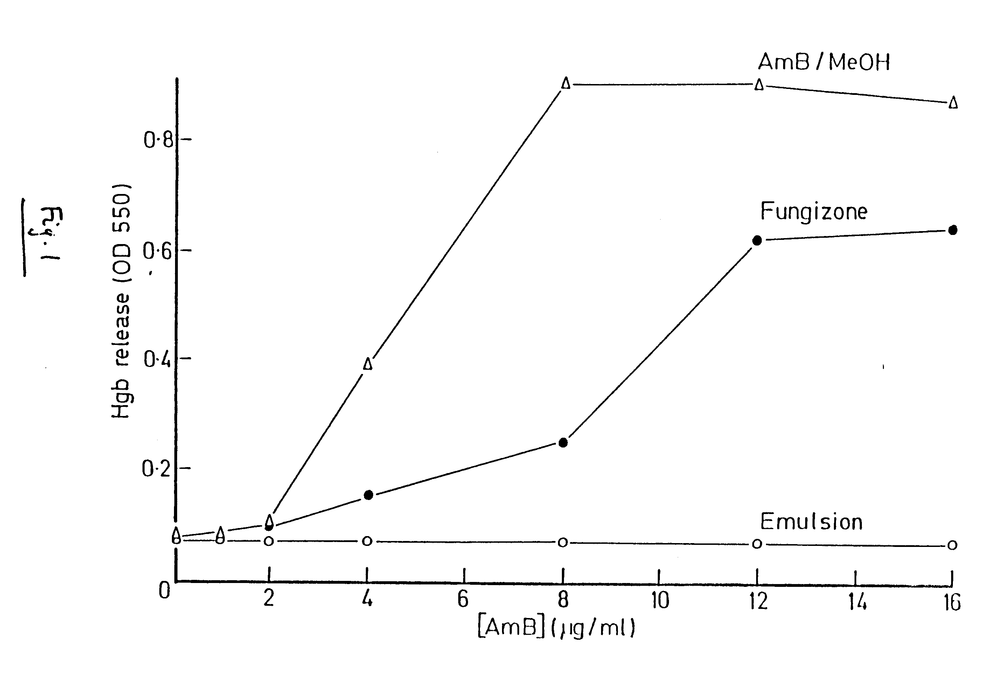

The lysis of human erythrocytes by the three formulations studied, as measured by haemoglobin release, is shown in Figure 1. Both Fungizone and methanolic solution of amphotericin cause cell lysis at amphotericin concentrations above 2 ug/ml. Lysis is total for both formulations above 12 ug/ml, since no intact cells could be sedimented from these solutions by centrifugation. Methanol alone (2%) did not cause detectable lysis.

The cells incubated with the emulsion formulation showed no detectable haemoglobin release.

The lack of erythrocyte lysis by the amphotericin emulsion in vitro indicates that the formulation has a lower toxicity to mammalian cells than Fungizone.

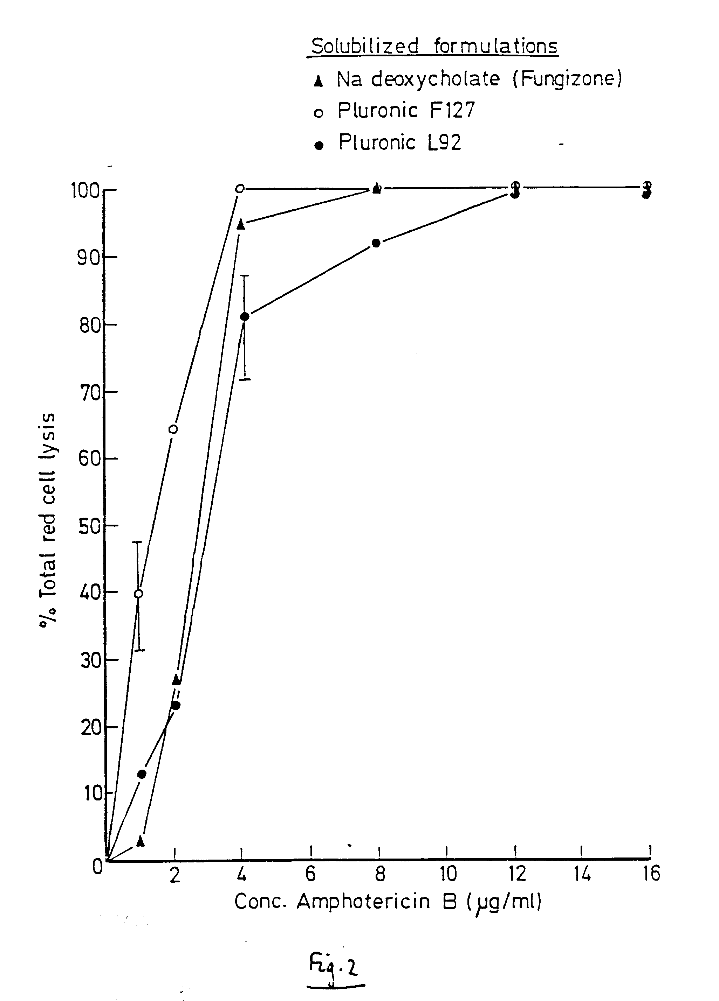

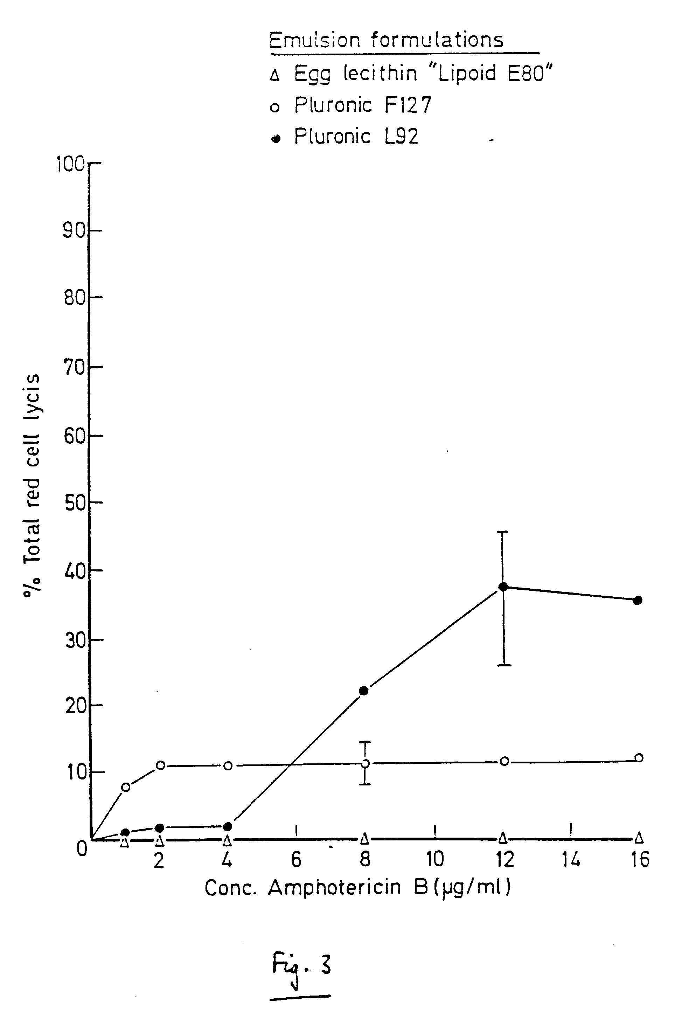

The emulsions prepared using the poloxamer materials Fl27 and L92 also demonstrated greatly reduced red cell lysis as compared to the commercial system Fungizone or to solubilized formulations using the copolymer alone without oil (Figure 2 and 3).

EXAMPLE 3: Ability to sterilise the emulsion without loss of amphotericin activity or emulsion stability

(a) Filtration

Emulsions of amphotericin prepared using an appropriate combination of oil (5-20% phase volume and nature, for example soybean oil, safflower oil, triolein with, if necessary, added monoglycerides and acetylated monoglycerides (Myvecet®)), and emulsifier concentration (egg lecithin 1-10% or block copolymer 1-10%) can be prepared using an ultra-efficient homogenisation procedure based upon the microfluidizer apparatus (Microfluidics Corporation, USA). The small resultant particle size, less than 200 nm average diameter, allows the emulsion to be passed through a terminal filter (220 nm) that renders the system stable.

(b) Heat sterilisation

Although amphotericin B is unstable to heat, it is possible to minimise the loss of the drug through the exploitation of the Fo concept together with the use of bactericidal materials. The Fo concept is an alternative approach to the classical pharmacopoeial conditions for sterilisation and is based upon the knowledge of bioloads within the formulation itself and those arising from pharmaceutical processing. It can be assumed that in a production process it will be possible to use a presterilised oil and emulsifier (minimum bioload). Furthermore, in the manufacturing process amphotericin B is exposed to methanol and this should also result in a minimisation of the bioload within the drug. Following from the above considerations, a sterilisation procedure comprising 121°C for 4 minutes in the presence of a bactericide should be sufficient to provide a sterile emulsion sample. Under this regimen the amphotericin demonstrated not more than 10% decomposition. It was noted that some of the amphotericin was precipitated from the emulsion during the sterilisation procedure in these early experiments. This precipitation phenomenon can be eliminated by the careful removal of the remaining methanol from the emulsion formulation, together with the addition of an antinucleating agent such as dextran. Such precipitation may result in an overestimate of the amount of amphotericin lost in the sterilisation procedure.

EXAMPLE 4: Long term stability

While the loss of amphotericin B during the heating cycle is of importance, the change in particle size of the emulsion during the sterilisation procedure and then the subsequent longer term stability of the drug and the physical stability of the emulsion have also been followed. UV techniques were used to follow drug loss. The techniques of photon correlation spectroscopy and laser diffractometry were used to follow emulsion particle size. Less than 10% loss of active component on storage and less than 10% change in mean diameter over at least 10 weeks was noted.

EXAMPLE 5: Stability

In further tests, emulsions in accordance with the invention were stored at 4°C. Amphotericin B was assayed by diluting the formulation into dimethyl sulphoxide and measuring absorbance at 415 nm. The emulsion droplet size was measured using a Malvern 2600 laser diffraction sizer. It should be noted that this sizer is insensitive to droplets of less than 1 micron diameter. This comprises the majority of droplets. The sizer quantifies the small proportion of large droplets produced by coalescence and is thus a sensitive indicator of emulsion stability. The true mean size of the emulsion (by photon correlation) is 200-350 nm.

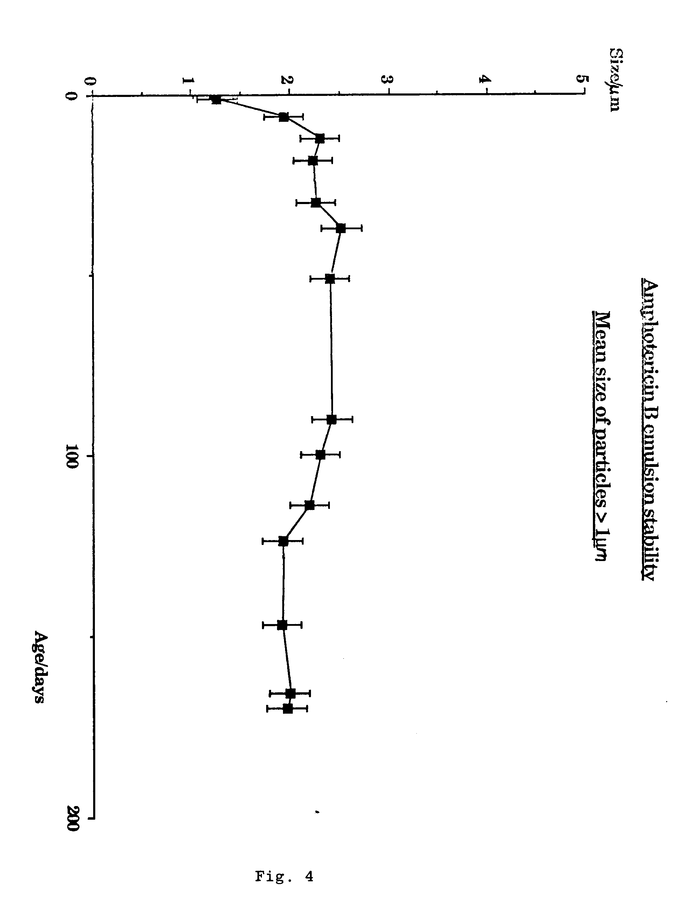

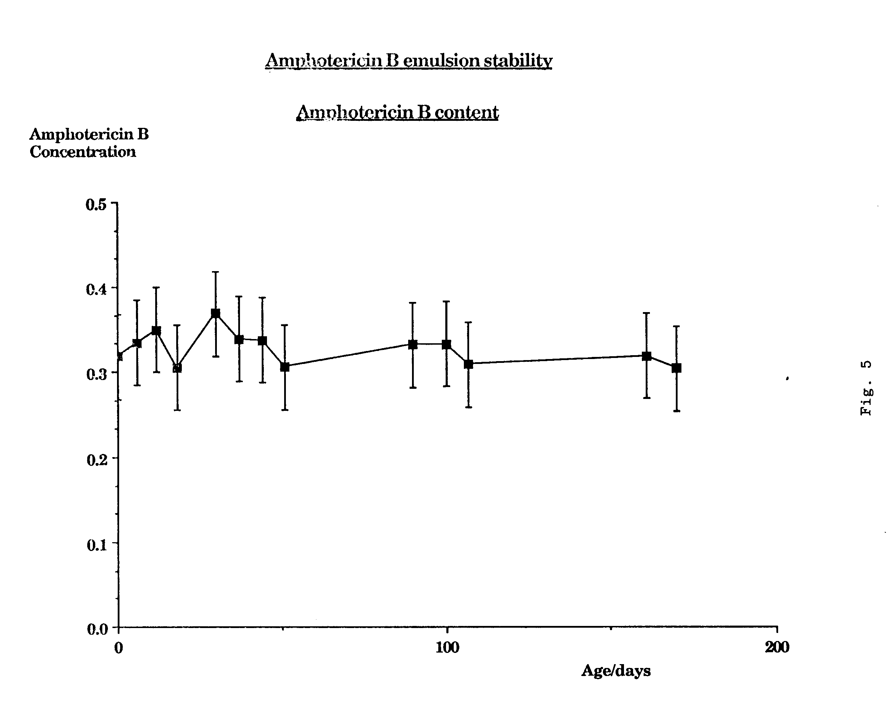

Figure 4 shows the stability of the particle sizes, and Figure 5 shows the stability of the amphotericin B content.

EXAMPLE 6: In vivo activity

Webster-derived CD-1 male mice (25g) from Charles River Laboratories were injected i.v. with approximately 1.2 x $10^6$ colony forming units of Candida albicans 3153A in saline for the establishment of experimental candidosis. This represented eight times the LD 50 and at this inoculum level 100% of the untreated animals died from a disseminated infection between 15-20 days post-inoculation. Infected animals were treated with either a formulation of the invention, Fungizone or virulence control formulation consisting of blank emulsion. Random groups of 10 infected mice were used for each drug level or for control. The preparations of AmB were given as a single injection i.v. 48 hours after infection. At this time fungal lesions were present in the kidneys, liver and brain of infected animals. Fungizone was administered in doses up to 4.0 mg/kg. Emulsion-associated AmB was administered in doses up to 9.0 mg/kg/ Survivors were recorded daily. The maximum tolerated dose (MTD) for Fungizone injected i.v. was 1.0 mg/kg in the infected mice. Emulsion associated AmB was tolerated at the highest dose administered (9.0 mg/kg).

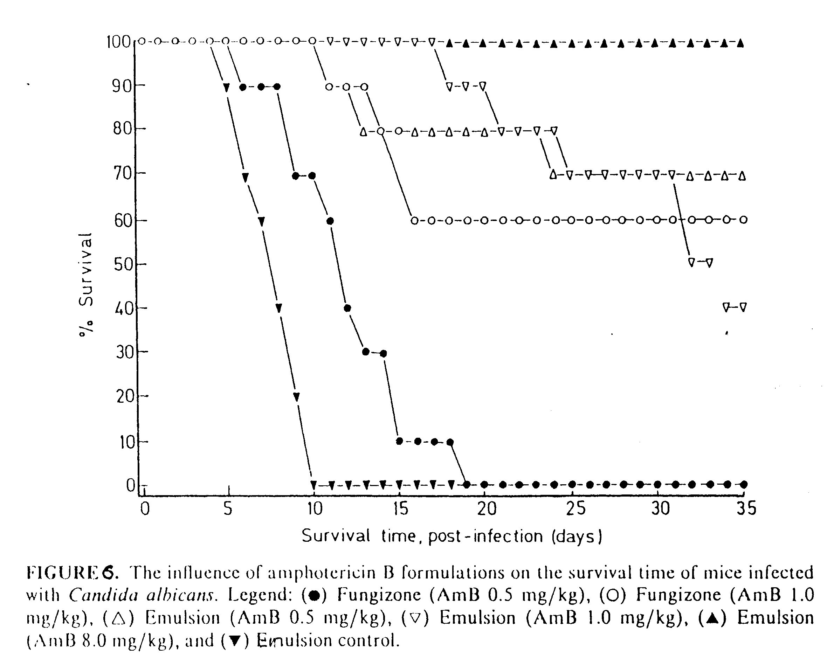

The effect of different formulations and concentrations of AmB on the survival of mice bearing established candidosis is shown in Figure 6. Eighteen days post infection with C. albicans 3153A, and eight days after the control animals had died, the ED50 values for Fungizone and the emulsion formulation were 0.78 mg/kg and less than 0.5 mg/kg, respectively, with 80% of the mice treated with the emulsion system surviving compared to the loss of all control animals 10 days after infection. Equally important, the reduced toxicity of the emulsion formulation (MTD) less than 8 mg/kg allowed the used of much higher doses of AmB such that at doses of 8 mg/kg all animals survived for 35 days as compared with only 60% survival during similar therapy with the much lower tolerated does of Fungizone.

## Claims

1. An oil-in-water surfactant-stabilised emulsion of a drug, the drug being poorly soluble in both oil and water, characterised in that at least 50% of the drug is present in the surfactant layer.

2. An emulsion according to Claim 1 wherein the said drug is a polyene antibiotic.

3. An emulsion according to Claim 2 wherein the drug is amphotericin B, nystatin or filipin.

4. An emulsion according to any of the preceding claims wherein the surfactant is lecithin or purified phosphatidyl choline.

5. An emulsion according to any of the preceding claims wherein, in the final emulsion, the proportion of oil is 5 to 50%.

6. An emulsion according to any one of the preceding claims wherein the surfactant is present at 0.5 to 10%.

7. A process for preparing an emulsion according to any of the preceding claims wherein the drug is solubilised in an aqueous preparation of the surfactant, such that at least 50% of the drug is present in the surfactant, before the emulsion is formed.

8. A process for preparing a drug-containing emulsion, comprising the steps of (a) optionally dissolving the drug in a suitable co-solvent, (b) mixing the drug or a solution thereof in the co-solvent with an aqueous preparation of a surfactant, (c) removing at least most of any co-solvent which is present, such that at least 50% of the drug is present in the surfactant, (d) mixing the product of step (c) with oil, and (e) forming an oil-in-water emulsion from the product of step (d).

9. A process according to Claim 8 wherein the said cosolvent is used.

10. A process according to Claim 8 wherein the cosolvent is methanol.

## Revendications

1. Emulsion de médicament huile-dans-eau stabilisée avec un agent tensio-actif, le médicament étant peu soluble tant dans l'huile que dans l'eau, caractérisé en ce qu'au moins 50% du médicament est présent dans la couche d'agent tensio-actif.

2. Emulsion selon la revendication 1 dans laquelle ledit médicament est un antibiotique à base de polyène.

3. Emulsion selon la revendication 2 dans laquelle le médicament est l'amphotéricine B, le nystatine ou la filipine

4. Emulsion selon l'une quelconque des revendications précédentes dans laquelle l'agent tensio-actif est la lécithine ou la phosphatidylcholine purifiée.

5. Emulsion selon l'une quelconque des revendications précédentes dans laquelle, dans l'émulsion finale, la proportion d'huile est de 5 à 50%.

6. Emulsion selon l'une quelconque des revendications précédentes dans laquelle l'agent tensio-actif est présent entre 0,5 et 10%.

7. Procédé de préparation d'une émulsion selon l'une quelconque des revendications précédentes dans lequel le médicament est solubilisé dans une préparation aqueuse de l'agent tensio-actif, de manière qu'au moins 50% du médicament soit présent dans l'agent tensio-actif avant que l'émulsion soit formée.

8. Procédé de préparation d'une émulsion contenant un médicament, comprenant les étapes de (a) dissolution facultative du médicament dans un co-solvant approprié, (b) mélange du médicament ou d'une solution du médicament dans le co-solvant avec une préparation aqueuse d'un agent tensio-actif, (c) élimination d'au moins la plus grande partie de tout co-solvant éventuellement présent, de manière qu'au moins 50% du médicament soit présent dans l'agent tensio-actif, (d) mélange du produit de l'étape (c) avec de l'huile, et (e) formation d'une émulsion huile-dans-eau à partir du produit de l'étape (d).

9. Procédé selon la revendication 8 dans lequel on utilise ledit co-solvant.

10. Procédé selon la revendication 8 dans lequel le co-solant est le méthanol.

**Patentansprüche**

1. Mit einem oberflächenaktiven Mittel stabilisierte Öl-in-Wasser-Emulsion eines Arzneimittels, wobei das Arzneimittel sowohl in Öl wie in Wasser schlecht löslich ist, dadurch gekennzeichnet, daß sich wenigstens 50% des Arzneimittels in der Schicht des oberflächenaktiven Mittels befinden.

2. Emulsion nach Anspruch 1, wobei das Arzneimittel ein Polyen-Antibiotikum ist.

3. Emulsion nach Anspruch 2, wobei das Arzneimittel Amphotericin B, Nystatin oder Filipin ist.

4. Emulsion nach einem der vorstehenden Ansprüche, wobei das oberflächenaktive Mittel Lecithin oder gereinigtes Phosphatidyl-cholin ist.

5. Emulsion nach einem der vorstehenden Ansprüche, wobei in der endgültigen Emulsion der Anteil des Öls 5 bis 50% ausmacht.

6. Emulsion nach einem der vorstehenden Ansprüche, wobei 0,5 bis 10% oberflächenaktives Mittel vorliegen.

7. Verfahren zur Herstellung einer Emulsion nach einem der vorstehenden Ansprüche, wobei das Arzneimittel in einer wäßrigen Präparation des oberflächenaktiven Mittels so solubilisiert wird, das wenigstens 50% des Arzneimittels in dem oberflächenaktiven Mittel vorliegen, bevor die Emulsion gebildet wird.

8. Verfahren zur Herstellung einer Arzneimittel-haltigen Emulsion, welches die Schritte umfaßt: (a) gegebenenfalls Lösen des Arzneimittels in einem geeigneten Co-Lösungsmittel, (b) Mischen des Arzneimittels oder einer Lösung davon in dem Co-Lösungsmittel mit einer wäßrigen Präparation eines oberflächenaktiven Mittels, (c) Entfernen zumindest des meisten Co-Lösungsmittels, welches vorliegt, so daß wenigstens 50% des Arzneimittels in dem oberflächenaktiven Mittel vorliegen, (d) Mischen des Produkts der Stufe (c) mit Öl, und (e) Bilden einer Öl-in-Wasser-Emulsion aus dem Produkt der Stufe (d).

9. Verfahren nach Anspruch 8, wobei das besagte Co-Lösungsmittel verwendet wird.

10. Verfahren nach Anspruch 8, wobei das Co-Lösungsmittel Methanol ist.

Fig. 1

EP 0 296 845 B1

Fig. 2

Fig. 3

Amphotericin B emulsion stability

Mean size of particles > 1µm

Fig. 4

Fig. 5

**FIGURE 6.** The influence of amphotericin B formulations on the survival time of mice infected with *Candida albicans*. Legend: (●) Fungizone (AmB 0.5 mg/kg), (○) Fungizone (AmB 1.0 mg/kg), (△) Emulsion (AmB 0.5 mg/kg), (▽) Emulsion (AmB 1.0 mg/kg), (▲) Emulsion (AmB 8.0 mg/kg), and (▼) Emulsion control.